# EUROPEAN PATENT SPECIFICATION

(11) **EP 0 533 090 B1**
(45) Date of publication and mention of the grant of the patent: **29.11.1995**
(21) Application number: 92115687.3
(22) Date of filing: 14.09.1992
(51) Int. Cl.: C07C 409/16, C08L 23/02, C08K 5/14

(54) **Liquid peroxidic compositions**
Flüssige Peroxidzusammensetzungen
Compositions peroxydiques liquides

(30) Priority: 19.09.1991 IT MI912491
(43) Date of publication of application: 24.03.1993
(73) Proprietor: ELF ATOCHEM ITALIA S.r.l., 20159 Milano (IT)
(72) Inventor: Merenda, Michele, I-15047 Alessandria (IT); De Franco, Rita, I-27058 Voghera (PV) (IT); Scotti, Carlo, I-27058 Voghera (PV) (IT)
(74) Representative: Simeoni, Lucio, Dr.

(56) References cited:
- EP-A- 0 101 175
- EP-A- 0 266 161
- FR-A- 2 379 518

## Description

The present invention refers to liquid peroxidic compositions of bis(alpha-t-butyl-peroxy-isopropyl)-benzene.

It is well known that the above compound, known as PEROXIMON F, is used in the vulcanization of polymers, e.g. polyethylene, elastomers, e.g. ethylene-propylene (EP) or containing also units deriving from a dienic monomer (EPDM) etc. See, for example, USP 3,764,628 and Italian Patent No. 651,288. But its use in the vulcanization of polymers is limited, being a compound solid at room temperature. In order to obtain a homogenous vulcanized compound it is therefore necessary to maintain PEROXIMON F in the molten state to have a uniform dosage.
This operation has the disadvantage, in particular, to impart losses in peroxidic content and the introduction of impurities.

It is known in the art to overcome these drawbacks by introducing additives having the function to lower the melting point under the room temperature.

However, this solution presents the drawback to reduce the efficiency of the vulcanization since the additives are inactive substances.

It is known from Italian Patent No. 1,209.130 to prepare liquid compositions of PEROXIMON F by solubilizing said bisperoxide with isopropylcumyl-t-butylperoxide. However the amount of cumylperoxide is very high, 75-90% by weight. Furthermore high quantities of cumylperoxide are required in order to obtain high MH values (see below) as requested in the vulcanization of the above-cited polymers, in particular polyethylene. This represents a drawback from the industrial viewpoint.

Further the composition is liquid at room temperature of 23°C. This is a drawback since it is desirable to have compositions which are liquid at room temperature up to 15°C so permitting a higher use range.

In this way it is avoided to melt the compound at a temperature lower than 23°C since this gives rise to the above-mentioned drawbacks as, for example, the introduction of impurities in the system which brings to a poor vulcanization in the finished article.

Other liquid peroxidic compositions are known from USP 4,202,790 and 4,239,644 where PEROXIMON F is substituted with DCP and is solubilized with cumylisopropyl-cumylperoxide or peroxides of similar formula. But also these peroxidic compositions present the drawback to be liquid only at 23°C, giving the same disadvantages said above.

From USP 4,450,302 liquid peroxidic compositions are also known, obtained from PEROXIMON F and DCP (dicumylperoxide) and t.butyl-cumyl-peroxide (PEROXIMON 166) which are liquid at very low temperatures, also lower than 5°C. Nevertheless these compositions present the drawback to be too volatile at a temperature of 15°C, due to the use of PEROXIMON 166.

Therefore it was desirable to have available liquid compositions of PEROXIMON F having the optimum balance of the following properties:
- to be liquid at room temperature, also at 15°C or lower;
- low volatility, so to permit that the formulates with the inert materials necessary for the vulcanization, as kaolin, calcium carbonate or master in elastomers or polymers in general, can be stocked for long periods without losses in peroxidic content;
- to show an optimum MH value (maximum value of the torque couple) combined with the optimum scorching time (see the definition below) such to obtain a high vulcanization and a uniform cross-linking.

In a preceding patent application the Applicant has surprisingly found that it is possible to obtain the combination of the above properties by the following composition consisting essentially of:
- from 65 to 95 parts by weight of bis(alpha-t-amyl-peroxy-isopropyl)-benzene (PEROXIMON 180) having formula: 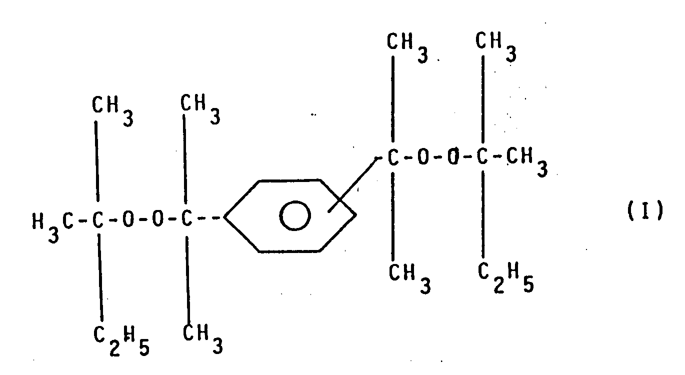
- from 5 to 35 parts by weight of bis(alpha-t-butyl-peroxy-isopropyl)-benzene (PEROXIMON F) of formula: 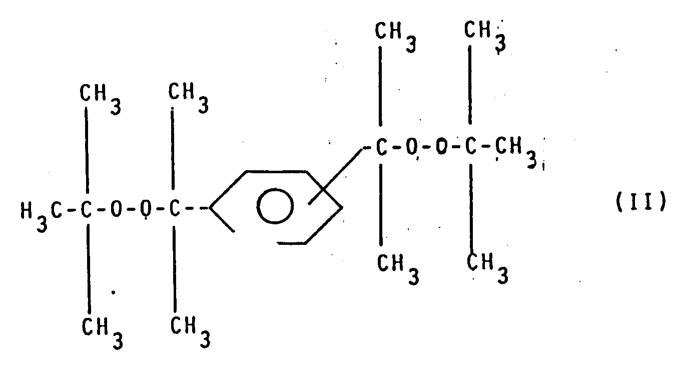
the substituting groups in (I) and (II) being in meta and/or para position with respect to the benzenic ring and excluding the combinations para PEROXIMON 180/para PEROXIMON F and metha PEROXIMON 180/para PEROXIMON F; preferably the meta/para ratios ranging between 1 and 2.0 in (I) and between 1.5 and 2.0 in (II) when mixtures of meta/para of (I) and (II) are used.

Liquid peroxidic compositions of (I) and (II) can be prepared by mixing (I) and (II) in the indicated ratios.

The Applicant has found that the cross-linking power of the indicated liquid compositions, at constant peroxidic content, increases proportionally with the increase of the ratio by moles terbutylic groups/(terbutylic groups + teramylic groups).

Up to a ratio of 0.27, the compositions are liquid at temperatures of 15°C and stable when stocked for a very long period.

The liquid compositions of the present invention show therefore a cross-linking power higher than PEROXIMON 180, which is liquid at room temperature but with a very low cross-linking power. Furthermore it is more advantageous from the economics to use the liquid compositions of the present invention since PEROXIMOIN F is obtained by cheaper reagents: t.butyl hydroperoxide instead of t-amyl hydroperoxide. Preparation processes of (I) and (II) are well known in the art, see, for example, USP 3,658,914 and Italian Patents No. 707,597, No. 651,288 and No. 805,543 in addition to ones indicated above.

A preferred process consists in the alkylation of benzene with an excess in propylene, the preferred molar ratio being 1:1.5, in the presence of AlCl₃ according to Friedel-Kraft reaction; if desirede, the mixture containing diisopropylbenzene, prevailingly metha and para, can be rectified to obtain the two isomers separated.

Then an oxidation is carried out by known methods, for example air, oxygen etc., in order to obtain the dihydroperoxide of the diisopropylbenzene.

The so prepared dihydroperoxide is extracted by adding an aqueous solution of caustic soda and a subsequent reduction according to known techniques, for instance by sulphides, sulfites, sulphydrates to obtain the dialcohol of formula: 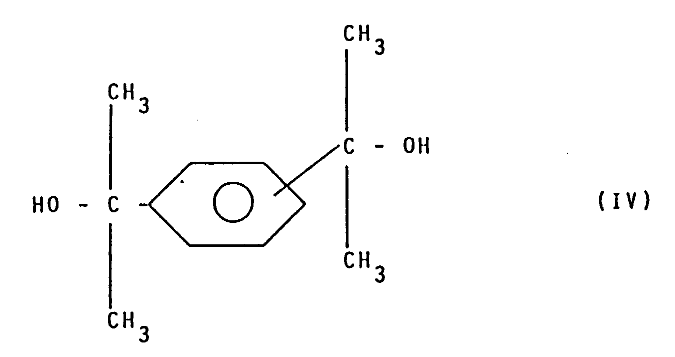
the two substituting groups of the benzenic ring being in metha and/or para position.

The alcohol is separated by centrifugation or filtration of the aqueous phase.

PEROXIMON F is then obtained by reacting the dialcohols (IV) with terbutylhydroperoxide, in hydrocarbons solution and/or diterbutylperoxide, or in aqueous solution at 70% by weight, in the presence of one of the known acid catalysts, among them e.g. the sulphuric, the metansulphonic, the paratoluensulphonic. The PEROXIMON F, product solid at room temperature, is obtained in metha and/or para form, depending on the starting alcohols.

The so obtained peroxidic mixture is washed, for example, by an alcaline solution; then it is concentrated to remove the low-boiling compounds, essentially hydrocarburic solvents and peroxidic by-products, such as diterbutylperoxide; preferably by distillation under steam current or by evaporaton of the solvent.

In a similar way the dialcohol (IV) can be reacted with teramylhydroperoxide, in hydrocarbons solution and/or in diteramylperoxide, so obtaining the PEROXIMON 180 (I).

However in its preceding patent application, the Applicant did not obtain very high MH values as it is desirable in the vulcanization of the above cited polymers; furthermore scorching times were not so high to permit a safe processing.

It has now surprisingly found that the above cited liquid compositions show a superior cross-linking power especially in PE (polyethylene) and superior scorching times when to the compositions of (I) and (II) is added also PEROXIMON 195, alpha(t-butyl-peroxy-isopropyl)alpha (t-amyl-peroxy-isopropyl)benzene, of formula: 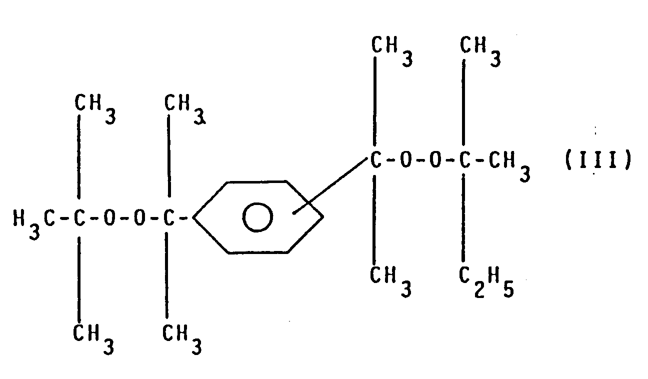
the two substituting groups being in meta and/or para position with respect to the benzenic ring.

It is therefore an object of the present invention to provide a ternary mixture containing:
- 10-55 parts by weight of (I);
- 5-35 parts by weight of (II);
- 30-55 parts by weight of (III), the meta/para isomers ratio being comprised preferably between 1.0 and 2.0 in all the components;

provided that when components (I), (II) and (III) are contemporaneously in the para form, component (I) is comprised between 30 and 55 and (II) between 5 and 15.

The above indicated advantages for the preceding patent application in the name of the Applicant are reached in a wider degree with the ternary mixtures of the present application.

It has unexpectedly found and forms a further object of the invention, a simplified process for the preparation of the ternary liquid compositions of the present invention, contemporaneously synthetizing the (I), (II) and (III) peroxides.

According to the process of the Applicant the variuos I, II and II bisperoxides can be contemporaneously obtained reacting the dialcohol (IV) with a mixture of tert.butylhydroperoxide and tert.amylhydroperoxide in a suitable range, from 3:2 to 1:4, by moles, in hydrocarbon solution and/or in ditert.butylperoxide and/or in ditert.amylperoxide, by operating in the presence of known acid catalysts among which, for instance, sulphuric, methansulphonic, paratoluensulphonic, so obtaining a mixture of PEROXIMON F, PEROXIMON 180 and PEROXIMON 195.

The obtained peroxidic solution is then washed with alcaline solutions and concentrated in analogous way as previously described.

The solution of tert.butylhydroperoxide and tert.amylhydroperoxide necessary for the simultaneous synthesis of the mixture containing PEROXIMON F, PEROXIMON 180, PEROXIMON 195 can be obtained by mixing the two hydroperoxides separately sinthetized according to already known techniques or synthetizing contemporaneously the two hydroperoxides.

According to this technique, already known for the synthesis of the single hydroperoxides, the two starting materials, tert.butyl alcohol and tert.amyl alcohol, are mixed in suitable ratio, from 3:2 to 1:4 by moles and then made to react with hydrogen peroxide in the presence of an acid catalyst, for example sulphuric acid, so obtaining a mixture of the two hydroperoxides in ditert.butylperoxide, ditert.amylperoxide, tert.butyl-tert.amylperoxide.

An hydrocarbon solvent can be added in the synthesis phase or after in order to obtain a solution containing the hydroperoxides at the necessary concentration for the synthesis of the liquid mixtures containing PEROXIMON 180, PEROXIMON F, PEROXIMON 195.

Similarly the hydroperoxides mixture can be obtained through contemporaneous absorption of isobutylene and isopentene in a sulphuric acid solution and reacting the mixture of esters with hydrogen peroxide or having the two olefin reactants above separately absorbed in sulphuric acid and mixing the two esters before reacting with hydrogen peroxide.

The three components I, II, III obtained by the process in situ can be separated for example by liquid chromathography to obtain the single pure components.

Another process for preparing PEROXIMON 195 is the following. It is reacted the meta/para monoalcohol monoperoxide of diisopropyl benzene (V) with tert.amyl hydroperoxide in hydrocarbon solution and/or ditert.amylperoxide, or in aqueous solution, in the presence of one of the known acid catalysts already cited. 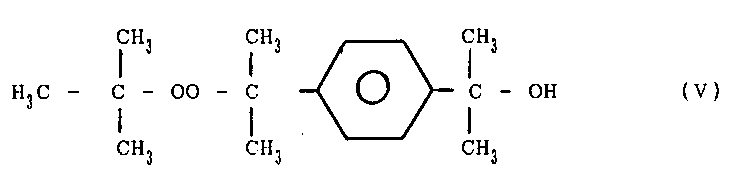
The monoalcohol monoperoxide (V) is obtained by reacting a strong excess of the dialcohol with tert.butylhydroperoxide in presence of sulphuric acid.

A further advantage of the ternary mixture with respect to the binary ones is a further decrease of the freezing point.

The cross-linking power of the ternary compositions tends to the one of PEROXIMON F and with the same active oxygen content it increases with the increase of the tert.butyl/tert.amyl groups ratio by moles and therefore with the increase of PEROXIMON F and PEROXIMON 195 content.

This is shown when considering the molar ratios terbutylic groups/(terbutylic + teramylic groups) with respect of the maximum value of the torque couple MH (which meaning is shown in example 1/d) obtained in the cross-linking tests of examples 1/d, 1/e, 3/c, 6/c and 7/c where peroxidic mixtures having the same active oxygen content are obtained.

It has been found that binary mixtures of PEROXIMON F/PEROXIMON 180, obtained by simple mixing of the two products, remain liquid at room temperature (15°C) up to a molar ratio terbutylic groups/sum of terbutylic and teramylic groups of 0.27 while the ternary mixtures of PEROXIMON F, PEROXIMON 180, PEROXIMON 195 remain liquid up to a molar ratio terbutylic groups/sum of terbutylic and teramylic groups of 0.59.

Therefore the use of such mixtures is more convenient than the use of PEROXIMON 180 alone, liquid too, both for the improved cross-linking power and for production cost being the raw material of PEROXIMON F and PEROXIMON 195, i.e. tert.butylhydroperoxide, cheaper than the raw material of PEROXIMON 180, i.e. tert.-amylhydroperoxide.

The polymers which can be cross-linked with the new compositions of the present invention are generally elastomeric polymers.

More particularly we can cite middle-, low-, high-density polyethylene, polybutene-1, ethylene/vinylacetate copolymers, acrylic esther/ethylene copolymers, ethylene/propylene copolymers, ethylene/butene-1 copolymers, ethylene/4-methyl-pentene-1 copolymers and propylene/butene-1 copolymers; moreover ethylene/propylene elastomeric polymers, EP or EPDM type, butylic rubber, chlorinated polyethylene and the propylene/butene-1 copolymer.

Also the mixture of two or more olefinic thermoplastic polymers, or two polymers of elastomeric type, or mixture of at least an elastomeric with a termoplastic polymer can be successfully cross-linked. The new compositions can be used not only in the vulcanization of compact articles, obtained by extrusion, but also for the manufacture of expanded articles (cross-linked) derived from the same materials, in particular polystyrene containing auto-extinguishing agents (flame-extinguishing agents); furthermore the same compositions can be used as coadjuvants polymers degradable by peroxides, for example polypropylene or poly-4-methyl-pentene-1, and as radicalic polymerization initiators.

Bisperoxides obtained by the cited processes can be purified by monoperoxides, present as by-products, according to the process of USP 3,584,059 which is entirely incorporated by reference in the present description.

In the examples: DUTRAL CO 054® means an E/P copolymer containing 42-48% by weight of propylene; RIBLENE CF 2203® mean a low-density polyethylene; ANOX HB® means 2,2,4-trimethyl-1,2-dihydrochinoline polymer.

The gaschromatographic analysis indicates the percentages by weight.

The following examples are given only with an illustrative but not limitative purpose of the present invention.

### EXAMPLE 1/a - Meta/para PEROXIMON 180 synthesis.

1 mole of benzol was alkylated with 1.5 moles of propyle ne, in the presence of AlCl₃ and according to usual reaction of Friedel-Kraft, so obtaining a mixture of benzene, cumene, diisopropylbenzenes, tri-isopropylbenzene.
The mixture was rectified to obtain meta-, para-diisopropylbenzene. This mixture was then oxidized by usual techniques with air so obtaining an oxidized mixture containing meta and para diisopropylbenzene, meta and para monohydroperoxide of the diisopropylbenzene, meta and para dihydroperoxide of the diisopropylbenzene.
The meta and para dihydroperoxide were extracted from said oxidized mixture by an aqueous solution of soda.
The extracted product was reduced with sodic sulphydrate, according to usual techniques, by this way all hydroperoxidic groups (-O-O-H) were transformed into alcoholic groups, so obtaining an aqueous suspension of the dialcohol (IV), constituted by 35% of the para isomer and by 65% of the meta isomer. 647 g of an eptanic solution containing 1.84 moles of teramylehydroperoxide were introduced in a glass-flask, equipped with stirrer and thermometer, then 174 g of the dialcohol (IV) containing 0.53 moles of the metha isomer, 0.28 moles of the para isomer and 0.97 moles of water were added.
Afterwards 331 g of an aqueous solution containing 70% by weight of sulphuric acid were gradually added during 60 minutes, at 5-8°C and under nitrogen stream then the solution was kept at 5-8°C for two hours under stirring.
Then the aqueous phase was separated in a separatory funnel, and two washings were effected with 500 cm³ each time (at 60°C) with an aqueous solution of soda at 10% by weight, then two washings followed with 200 cm³ each time of deionized water.
After elimination of the heptane under steam current and after anhydrification under vacuum at 65°C, 193 g of a peroxidic mixture were obtained having an active oxygen content equal to 6.97% by weight, a purity degree of 81% by weight (as total peroxidic content), a density of 0.933 g/cm³ and a viscosity (at 25°C) of 97 mPa.s.
The half-points of the mixture (by thermic decomposition) were 107°C (at 10 hours) and 178°C (at 1 minute); the gas-chromatographic analysis showed the following composition:

| | |
|---|---|
| - meta PEROXIMON 180 | 45% by weight |
| - para PEROXIMON 180 | 36% by weight. |

The rest were by-products.

### EXAMPLE 1/b - Meta PEROXIMON 180 synthesis.

644 g of an heptanic solution containing 1.84 moles of teramylehydroperoxide were introduced in a glass-flask, equipped with stirrer and thermometer, then 174 g of dialcohol of formula (IV) were added, containing 0.807 moles of the pure metha isomer and 0.98 moles of water.
Then 331 g of an aqueous solution containing 70% by weight of sulphuric acid were gradually added, during 60 minutes, at 5-8°C and under nitrogen stream; and the solution was kept at 5-8°C for two hours under stirring.
Then the aqueous phase was separated in a separating flutter and two washings were effected, with 500 cm³ each time (at 60°C) of an aqueous solution of soda at 10% by weight, followed by other two washings with 200 cm³ each time of deionized water.
After elimination of heptane under steam current, and after anhydrification under vacuum at 65°C, 178 g of peroxidic mixture were obtained having an active oxygen content of 7.01% by weight, a purity degree of 81% by weight (as total peroxidic content), a density of 0.931 g/cm³ and a viscosity (at 25°C) of 74.47 mPa.s.
The rest were by-products.

### EXAMPLE 1/c - Para PEROXIMON 180 synthesis.

644 g of an heptanic solution containing 1.84 mole of teramylehydroperoxide were introduced in a glass-flask, equipped with stirrer and thermometer, then 174 g of the dialcohol of formula (IV) were added, containing 0.807 mole of pure para isomer and 0.98 moles of water.
Then 331 g of an aqueous solution containing 70% by weight of sulphuric acid were gradually added, during 60 minutes, at 5-8°C under nitrogen stream, and the solution was maintained at 5-8°C for two hours under stirring.
Then the aquesous phase was separated in a separating funnel and two washings were effected, with 500 cm ³ each time (at 60°C) of aqueous solution of soda at 10% by weight, followed by other two washings with 200 cm³ each time of deionized water.
After heptane elimination under steam current, and after anhydrification under vacuum at 65°C, 202 g of peroxidic mixture were obtained with an active oxygen content equal to 7.02% by weight, a purity degree of 81% by weight (as total peroxidic content), a density of 0.935 g/cm³ arid a viscosity (at 25°C) di 109.1 mPa.s.
The rest were by-products.

### EXAMPLE 1/d (comparison test)

### Applicative test of meta/para PEROXIMON 180.

100 parts by weight of an ethylene-propylene elastomeric copolymer, commercially known as DUTRAL CO 054®, were mixed with 0.3 parts by weight of sulphur, 5 parts by weight of ZnO and 50 parts by weight of carbon black.
2.26 parts by weight of the peroxidic mixture of example 1/a were added and the resulting mixture was homogenized in a calender for a time of 5 minutes; the product coming from the calender showed the following properties:
a) ODR curve data at 170°C (oscillation arc = 3°; oscillation frequency 100 cycles/minute):

| | |
|---|---|
| MH | = 74.5 pounds * inches |
| ts10 | = 150 seconds |
| t90 | = 672 seconds |

The ODR curve (Oscillating Disc Rheometer) can be obtained by the aid of a rotating disc rheometer, under the ASTM-D-2084-71T rules.
The times are reported in ascissa and in ordinates the torque couple (pounds.inches) measured by dinamometer, opposed by the polymer to the rotation of the disk. It was found that the maximum reticulation density had a couple maximum value (MH = 74.5 pounds.inches) which no more changed with the time.
The expressions t90 and ts10 mean respectively the time occurring to reach a twisting couple equal to 90% of the maximun torque couple and the time occurring to reach a level of 10 pounds.inches over the ODR curve minimum.
b) "Scorching" times at Mooney viscosimeter (135°C):

| | |
|---|---|
| t2 | = 588 seconds |
| t5 | = 930 seconds |

By "scorching" it is intended the undesired premature vulcanization occurring during the compound extrusion, before the exit from the extruder die; said premature vulcanization often causes an interruption of the operations.
For scorching time t2 or t5 (at Mooney viscosimeter) it is intended the time occurring to reach an increase of the minimum viscosity value equal to 2 or to 5 Mooney units respectively.
The viscosity was determined by a Mooney viscosimeter at cutting disc (ASTM D 1646-81 rules).

### EXAMPLE 1/e (comparison test)

### Applicative test meta/para PEROXIMON 180.

100 parts by weight of polyethylene, commercially known as RIBLENE CF 2203® were mixed with 0.5 parts by weight of ANOX HB® and 0.5 parts by weight of TAC (triallyl cyanurate). 2.19 parts by weight of the peroxidic mixture of example 1/a were added and the resulting mixture was homogenized in a calender, the product of the calender showed the following properties:
a) ODR curve data at 170°C (oscillation arc = 1°)

| | |
|---|---|
| MH | = 16.93 pounds * inches |
| ts10 | = 135 seconds |
| t90 | = 950 seconds |

b) "Scorching" times at Mooney viscosimeter (135°C):

| | |
|---|---|
| t5 | = 1034 seconds |
| t10 | = 1236 seconds. |

### EXAMPLE 2 (comparison test)

### Applicative example of binary mixtures PEROXIMON 180/PEROXIMON F

### Example 2/a

100 parts by weight of an elastomeric ethylene-propylene copolymer, commercially known as DUTRAL CO 054® were mixed with 0.3 parts by weight of sulphur, 5 parts by weight of ZnO and 50 party by weight of carbon black.
2.20 parts by weight of the peroxidic mixture containing 80% by weight of PEROXIMON 180 meta/para and 20% by weight of PEROXIMON F meta/para were added arid the resulting compound was homogenized in a calender; the product of the calender showed the following properties:
a) ODR curve data at 170°C (oscillation arc = 3°; oscillation frequency 100 cycles/minute).

| | |
|---|---|
| MH | = 78.1 pounds * inches |
| ts10 | = 140 seconds |
| t90 | = 699 seconds. |

b) "Scorching" times at Mooney viscosimeter (135°C):

| | |
|---|---|
| t2 | = 590 seconds |
| t5 | = 890 seconds. |

### Example 2/b

100 party by weight of polyethyelne, commercially known as RIBLENE CF 2203® were mixed with 0.5 parts by weight of ANOX HB® and 0.5 parts by weight of TAC (triallyl cyanurate). 2,16 party by weight of the peroxidic mixture of example 2/a were added and the resulting compound was homogenized in a calender, the product of the calender showed the following properties:
a) ODR curve data at 170° (oscillation arc = 1°)

| | |
|---|---|
| MH | = 16.99 pounds * inches |
| ts10 | = 136 seconds |
| t90 | = 952 seconds |

b) Scorching times at Mooney viscosimeter (135°):

| | |
|---|---|
| t5 | = 1038 seconds |
| t10 | = 1240 seconds |

### EXAMPLE 3/a - Meta-para (PEROXIMON 180/PEROXIMON F/PEROXIMON 195) contemporaneous synthesis.

138 g of an heptanic solution containing 0.26 moles of terbutyl hydroperoxides and 0.26 moles of teramyl hydroperoxide were introduced in a glass flask, equipped with stirrer and thermometer; then 56 g of dialcohol (IV) were added, containing 0.167 moles of the meta isomer, 0.089 moles of the para isomer and 0.34 moles of water.
119 g of an aqueous solution containing 70% by weight of sulphuric acid were added gradually, during 60 minutes at 5-8°C and under nitrogen stream, and the solution was maintained at 5-8°C for 1 hour under stirring.
At the end of the reaction the aqueous phase was separated in a separating funnel and two washings were effected, with 250 cm³ each time of an aqueous solution of soda at 10% by weight at 60°C, followed by other two washings with 200 cm³ each time of deionized water.
After heptane elimination by distillation under steam current and after anhydrification under vacuum at 65°C, 67 g of peroxidic mixture were obtained with an active oxygen content of 7.43% by weight, a density of 0.922 g/cm³ and a viscosity (at 35°C) of 64.70 mPa.s.
The half-points of the mixture (by thermic decomposition) were 113°C (at 10 hours) and 182°C (at 1 minute).
The gas chromatographic analysis showed the following composition:

| | |
|---|---|
| meta PEROXIMON F | 19% by weight |
| para PEROXIMON F | 12% by weight |
| meta PEROXIMON 180 | 8% by weight |
| para PEROXIMON 180 | 7% by weight |
| meta PEROXIMON 195 | 26% by weight |
| para PEROXIMON 195 | 20% by weight |

The rest were by-products.
The product was liquid up to 15°C.

### EXAMPLE 3/b - Meta (PEROXIMON 180/PEROXIMON F/PEROXIMON 195) contemporaneous synthesis.

138 g of an heptanic solution containing 0.26 terbutyl hydroperoxides moles and 0.26 teramyl hydroperoxide moles were introduced in a glass flask, equipped with stirrer and thermometer; then 56 g of dialcohol (IV) were added, containing 0.26 moles of the pure metha isomer and 0.34 moles of water.
119 g of an aqueous solution containing 70% by weight of sulphuric acid were added gradually, during 60 minutes at 5-8°C under nitrogen stream, and the solution was maintained at 5-8°C for 1 hour under stirring.
The acqueous phase was separated in a separating funnel and two washings were effected with 250 cm³ each time at 60°C with an aqueous solution of soda at 10% by weight followed by other two washings with 200 cm³ each time of deionized water.
After heptane elimination by distillation under steam current and after anhydrification under vacuum at 65°C, 60 g of peroxidic mixture were obtained with an active oxygen content of 7.8% by weight, a density of 0.916 g/cm³ and a viscosity (at 25°C) of 52.73 mPa.a.
The gas chromatographic analysis showed the following composition:

| | |
|---|---|
| meta PEROXIMON F | 30% by weight |
| meta PEROXIMON 180 | 16% by weight |
| meta PEROXIMON 195 | 43% by weight |

The rest were by-products.
The product was liquid at room temperature of 15°C.

### EXAMPLE 3/c - Applicative test of meta/para (PEROXIMON 180/PEROXIMON F/PEROXIMON 195) mixture.

100 parts by weight of an ethylene-propylene elastomeric copolymer commercially known as DUTRAL CO 054® were mixed with 0.3 parts by weight of sulphur, 5 parts by weight of ZnO and 50 party by weight of carbon black.
2.12 parts by weight of the peroxidic mixture of example 3/a were added and the resulting compound was homogenized in a calender; the product of the calender showed the following properties:
a) ODR curve data at 170° (oscillation arc = 3°; oscillation frequency 100 cycles/minute).

| | |
|---|---|
| MH | = 88.5 pounds * inches |
| ts10 | = 132 seconds |
| t90 | = 810 seconds |

b) Scorching times at Mooney viscosimeter (135°C):

| | |
|---|---|
| t2 | = 582 seconds |
| t5 | = 852 seconds. |

### EXAMPLE 4/a - Meta/para (PEROXIMON F/PEROXIMON 180/PEROXIMON 195) mixture contemporaneous synthesis.

128 g of an heptanic solution containing 0.13 terbutyl hydroperoxides moles and 0.39 teramyl hydroperoxide moles were introduced in a glass flask equipped with stirrer and thermometer, then 56 g of dialcohol (IV) were added, containing 0.167 moles of the meta isomer, 0.089 moles of the para isomer and 0.34 moles of water.
119 g of an aqueous solution containing 70% by weight of sulphuric acid were added gradually, during 60 minutes, at 5-8°C under nitrogen stream, and the solution was maintained at 5-8°C for 60 minutes under stirring.
At the end of the reaction the aqueous phase was separated in a separating funnel and two washings were effected with 250 cm³ each time (at 60°C) of aqueous solution of soda at 10% by weight, followed by other two washings with 200 cm³ each time of deionized water.
After heptane elimination by distillation in steam current and after anhydrification under vacuum at 65°C, 66 g of a peroxidic mixture were obtained with an active oxygen content of 7.27% by weight, a density of 0.937 g/cm³ and a viscosity (at 25°C) of 65 mPa.s.
The half-points of the mixture by thermic decomposition were 110°C (at 10 hours) and 180°C (at 1 minute).
The gas chromatographic analysis showed the mixture contents:

| | |
|---|---|
| meta PEROXIMON F | 6% by weight |
| para PEROXIMON F | 3% by weight |
| meta PEROXIMON 180 | 24% by weight |
| para PEROXIMON 180 | 19% by weight |
| meta PEROXIMON 195 | 22% by weight |
| para PEROXIMON 195 | 15% by weight. |

The rest were by-products.
The product was liquid at room temperature of 15°C.

### EXAMPLE 4/b - Meta (PEROXIMON F/PEROXIMON 180/PEROXIMON 195) mixture contemporaneous synthesis.

124 g of an heptanic solution containing 0.124 terbutylhydroperoxide moles and 0.373 teramylhydroperoxide moles were introduced in a glass flask, equipped with stirrer and thermometer; then 54 g of dialcohol (IV) were added, containing 0.245 moles of meta isomer only and 0.34 moles of water.
119 g of an aqueous solution containing 70% by weight of sulphuric acid were added gradually, during 60 minutes, at 5-8°C under hydrogen steam, and the solution was maintained a 5-8°C for 60 minutes under stirring.
At the end of the reaction the aqueous phase was separated in a separating funnel and two washings were effected, with 250 cm³ each time (at 60°C) of an acqueous solution of soda at 10% by weight, followed by other two washings with 200 cm³ each time of deionizwed water.
After heptane elimination by distillation in steam current and after anhydrification under vacuum ay 65°C, 56 g of a peroxidic mixture were obtained with an active oxygen content of 7.62% by weight, a density of 0.911 g/cm³ and a viscosity (at 25°C) of 66.7 mPa.s.
The gas chromatographic analysis showed the following composition:

| | |
|---|---|
| meta PEROXIMON F | 9% by weight |
| meta PEROXIMON 180 | 43% by weight |
| meta PEROXIMON 195 | 37% by weight |

The rest were by-products.
The product was liquid at room temperature of 15°C.

### EXAMPLE 4/c - Para (PEROXIMON F/PEROXIMON 180/PEROXIMON 195) mixture contemporaneous synthesis.

130 g of an heptanic solution containing 0.124 terbutylhydroperoxide moles and 0.373 teramylhydroperoxide moles were introduced into a glass flask, equipped with stirrer and thermometer, then 56 g of dialcohol (IV) were added, containing 0.246 moles of pure para isomer and 0.34 moles of water.
119 g of an aqueous solution containing 70% by weight of sulphuric acid were added gradually, during 60 minutes, at 5-8°C under nitrogen steam, and the solution was left at 5-8°C for 60 minutes under stirring.
At the end of the reaction the acqueous phase was separated in a separating funnel and two washings were effected with 250 cm³ each time (at 60°C) of an aqueous solution of soda at 10% by weight, followed by other two washings with 200 cm³ each time of deionized water.
After heptane elimination by distillation under steam current and after anhydrification under vaccum at 65°C, 66 g of a peroxidic mixture were obtained with an active oxygen content of 8.1% by weight, a density of 0.918 g/cm³ and a viscosity (at 25°C) of 106.6 mPa.s.
The gas chromatographic analysis showed the following composition:

| | |
|---|---|
| para PEROXIMON F | 7% by weight |
| para PEROXIMON 180 | 47% by weight |
| para PEROXIMON 195 | 38% by weight |

The rest were by-products.
The product was liquid at room temperature of 15°C.

### EXAMPLE 5 - Applicative test meta/para (PEROXIMON 180/PEROXIMON F/PEROXIMON 195)

100 parts by weight of an ethylene-propylene elastomeric copolymer, commercially known as DUTRAL CO 054 ^{R} were mixed with 0.3 parts by weight of sulphur, 5 parts by weight of ZnO and 50 parts by weight of carbon black.
2.22 parts by weight of the peroxidic mixture of example 4/a were added and the resulting compound was homogenized in a calender; the product of the calender showed the following properties:
a) ODR curve data at 170°C (oscillation arc = 3°; oscillation frequency 100 cycles/minute)

| | |
|---|---|
| MH | = 82 pounds * inches |
| ts10 | = 138 seconds |
| t90 | = 732 seconds. |

b) Scorching times at Mooney viscosimeter (135°C):

| | |
|---|---|
| t2 | = 570 seconds |
| t5 | = 852 seconds |

### EXAMPLE 6/a - Contemporaneous synthesis of the mixture containing terbutyl-teramyl hydroperoxide.

84.2 g of 88% terbutylic alcohol (the rest consisting of water) industrially produced, 100 g af 88% teramylic alcohol (the rest consisting of water), 197 g of n-heptane were mixed in a glass flask, equipped with stirrer and thermometer; successively, 126 g of 70% hydrogen peroxide and 126 g of 70% by weight sulphuric acid were added gradually and contemporaneously, during 60 minutes at a temperature of 35°C; the reaction mass was kept under stirring for 120 minutes always at 35°C.
At the end of the reaction, the acqueous phase containing the sulphuric acid and the hydrogen peroxide in excess was separated from the organic phase containing heptane, teramylhydroperoxide and terbutylhydroperoxide.
386 g of an organic solution were obtained, showing the following composition at gas chromatographic analysis:

| | |
|---|---|
| terbutyl hydroperoxide | 20.7% |
| teramyl hydroperoxide | 23.9% |

the rest was heptane and reaction by-products.

### EXAMPLE 6/b - Contemporaneous synthesis of the mixture containing meta/para (PEROXIMON F/PEROXIMON 195/PEROXIMON 180) by using the hydroperoxides mixture of example 6/a.

113 g of the heptanic solution obtained by example 6/a, containing 0.26 moles of terbutylhydroperoxide and 0.26 moles of teramylhydroperoxide were introduced in a glass flask, equipped with stirrer and thermometer, then 56 g of dialcohol (IV) were added, containing 0.167 moles of meta isomer, 0.089 moles of para isomer and 0.34 moles of water.
119 g of an aqueous solution containing 70% by weight of sulphuric acid were added gradually, during 60 minutes, at 5-8°C under nitrogen stream, and the solution was maintained at 5-8°C for 60 minutes under stirring.
At the end of the reaction the aqueous phase was separated in a separating funnel and two washings were effected, with 250 cm³ each time (at 60°C) of aqueous solution of soda at 10% by weight, followed by other two washings with 200 cm³ each time of deionized water.
After heptane elimination by distillation under steam current and after anhydrification under vacuum at 65°C, 66 g of product were obtained having an active oxygen content of 7.53% by weight, a density of 0.920 g/cm³ and a viscosity (at 25°C) of 64.7 mPa.s.
The gas-chromatographic analysis showed the following composition:

| | |
|---|---|
| meta PEROXIMON F | 15% by weight |
| para PEROXIMON F | 11% by weight |
| meta PEROXIMON 180 | 9% by weight |
| para PEROXIMON 180 | 8% by weight |
| meta PEROXIMON 195 | 25% by weight |
| para PEROXIMON 195 | 21% by weight |

The rest were by-products.
The product was liquid at room temperature of 15°C.

### EXAMPLE 6/c - Applicative test meta/para (PEROXIMON 180/PEROXIMONF/PEROXIMON 195) mixture.

100 parts by weight of polyethylene, commercially known as RIBLENE CF 2203® were mixed with 0.5 parts by weight of ANOX HB® and 0.5 parts by weight of TAC (triallyl cyanurate). 2.11 parts by weight of the peroxidic mixture of example 6/b were added and the resulting compound was homogenized in a calender, the product of the calender showed the following properties:
a) ODR curve data at 170°C (oscillation arc = 1°)

| | |
|---|---|
| MH | = 19.07 pounds * inches |
| ts10 | = 126 seconds |
| t90 | = 963 seconds |

b) Scorching times at Mooney viscosimeter (135°C):

| | |
|---|---|
| t5 | = 1437 seconds |
| t10 | = 1740 seconds |

### EXAMPLE 7/a - Contemporaneous synthesis of the mixture containing terbutyl-teramyl hydroperoxide.

21 g of 88% terbutylic alcohol (the rest consisted of water), 75 g of 88% teramylic alcohol (the rest consisted of water), 100 g of n-heptane were mixed in a glass flask, equipped with stirrer and thermometer; successively, 63 g of 70% by weight aqueous solution of hydrogen peroxide arid 63 g of 70% by weight of an aqueous solution of sulphuric acid were added gradually and contemporarily, during 60 minutes at a temperature of 35°C; the reaction mass was left under stirring for 120 minutes at a temperature of 35°C.
At the end of the reaction the acqueous phase containing the sulphuric acid and the hydrogen peroxide in excess was separated from the organic phase containing heptane, teramyl hydroperoxide, terbutyl hydroperoxide.
199 g of an organic solution showing the following gas-chromathographic composition were obtained:

| | |
|---|---|
| terbutyl hydroperoxide | 10% |
| teramyl hydroperoxide | 30% |

The rest was heptane and by-products.

### EXAMPLE 7/b - Oontemporaneous synthesis of the mixture containing meta/para (PEROXIMON F/PEROXIMON 195/PEROXIMON 180) by using the hydroperoxides mixture of Example 7/a.

130 g of the heptanic solution obtained in example 7/a, containing 0.144 moles of terbutylhydroperoxide and 0.375 moles of teramyl hydroperoxide, were introduced in a glass flask equipped with stirrer and thermometer, then 56 g of dialcohol (IV) were added, containing 0.167 moles of meta isomer, 0.089 moles of para isomer and 0.34 moles of water.
119 g of an aqueous solution containing 70% by weight of sulphuric acid were added gradually, during 60 minutes, at 5-8°C and under nitrogen stream, and the solution was left always at 5-8°C for 60 minutes under stirring.
At the end of the reaction the aqueous phase was separated in a separating funnel and two washings were effected with 250 cm³ each time (at 60°C) of an aqueous solution of soda at 10% by weight, followed by other two washings with 200 cm³ each time of deionized water.
After heptane elimination by distillation under steam current and after anhydrification under vaccum at 65°C, 67 g of a peroxidic mixture were obtained with an active oxygen content of 7.27% by weight, a density of 0.935 g/cm³ and a viscosity (at 25°C) of 64.8 mPa.s.
The gas-chromatographic analysis showed the following composition:

| | |
|---|---|
| meta PEROXIMON F | 5% by weight |
| para PEROXIMON F | 3% by weight |
| meta PEROXIMON 180 | 23% by weight |
| para PEROXIMON 180 | 19% by weight |
| meta PEROXIMON 195 | 21% by weight |
| para PEROXIMON 195 | 17% by weight |

The rest were by-products.
The product was liquid at room temperature of 15°C.

### EXAMPLE 7/c - Applicative test meta/para (PEROXIMON 180/PEROXIMON F/PEROXIMON 195) mixtures.

100 parts by weight of polyethylene, commercially known as RIBLENE CF 2203® were mixed with 0.5 parts by weight of ANOX HB® and 0.5 parts by weight of TAC (triallyl cyanurate). 2.19 parts by weight of the peroxidic mixture of example 7/b were added and the resulting composition was homogenized in a calender, the product coming from the calender showed the following properties:
a) ODR curve data at 170°C (oscillation arc = 1°)

| | |
|---|---|
| MH | = 17.54 pounds * inches |
| ts10 | = 140 seconds |
| t90 | = 1097 seconds |

b) Scorching times at Mooney viscosimeter (135°C):

| | |
|---|---|
| t5 | = 1437 seconds |
| t10 | = 1751 seconds |

From the above data the results obtained by polyethylene show an optimal balance of the above-reported properties.

### EXAMPLE 8/a - Synthesis of monoalcohol monoperoxide of meta/para diisopropyl benzene.

184 g of an heptanic solution containing 0.48 moles of terbutyl hydroperoxide were introduced in a glass-flask, equipped with stirrer and thermometer. Then 336 g of dialcohol of formula (IV) were added, containing 1.013 moles of the meta isomer, 0.54 g of the para isomer; then 1.86 moles of water were added.
Afterwards 88 g of an aqueous solution containing 70% by weight of sulphuric acid were gradually added during 20 minutes, at 5-8°C and under nitrogen stream. Then the solution was kept at 5-8°C for 30 minutes under stirring.
Then the unreacted solid dialcohol was separated by filtration.
Then the aqueous phase was separated from the organic one by means of a separatory funnel, and the latter was washed with 200 cm³ of deionized water.
187 g of a peroxidic mixture were obtained. The gas-chromatographic analysis showed the following composition:

| | |
|---|---|
| meta monoalcohol monoperoxide | 8.8% by weight |
| para monolacohol monoperoxide | 25.9% by weight |

The rest were heptane and by-products.

### EXAMPLE 8/b - Synthesis of PEROXIMON 195 meta/para.

187 g of heptanic solution of Example 8/a of formula (V) containing 0.062 moles of meta isomer and 0.182 moles of para isomer were introduced in a glass-flask, equipped with stirrer and thermometer; then 65 g of an heptanic solution containing 0.3 moles of teramyl hydroperoxide were added.
88 g of an aqueous solution containing 70% by weight of sulphuric acid were gradually added, in 60 minutes, at 5-8°C and under nitrogen stream; after the solution was kept at 5-8°C under stirring for 60 minutes.
Then the aqueous phase was separated from the organic one by means of separatory funnel, and the latter was washed with 500 cm³ (at 60°C) of an aqueous solution of soda at 10% by weight, followed by other washing with 200 cm³ of deionized water.
After elimination of heptane under stream current, and after anhydrification under vacuum at 65°C, 85 g of peroxidic mixture were obtained having an active oxygen content of 7.3% by weight, a purity degree of 81% by weight (as total peroxidic content).
The gas-chromastographic analysis showed the following composition:

| | |
|---|---|
| meta PEROXIMON 195 | 20.47% by weight |
| para PEROXIMON 195 | 60.29% by weight |

The rest were by-products.

### EXAMPLE 8/c - Applicative test of meta/para PEROXIMON 195.

100 parts by weight of polyethylene, commercially known as RIBLENE CF 2203® were mixed with 0.5 parts by weight of ANOX HB® and 0.5 parts by weight of TAC (triallyl cyanurate). 2.19 parts by weight of the peroxidic mixture of Example 8/b were added and the resulting composition was homogenized in a calender, the product coming from the calender showed the following properties:
a) ODR curve data at 170°C (oscillation arc = 1°)

| | |
|---|---|
| MH | = 18.57 pounds * inches |
| ts10 | = 143 seconds |
| t90 | = 1065 seconds. |

b) Scorching times at Mooney viscosimeter (135°C):

| | |
|---|---|
| t5 | = 930 seconds |
| t10 | = 1160 seconds |

## Claims

1. Liquid peroxidic compositions constituted by the following components per 100 parts by weight of composition:
- from 10 to 55 parts by weight of bis(alpha-t-amylperoxy-isopropyl)-benzene (I), the two substituents being in meta and/or para position with respect to the benzene ring, the meta/para ratio ranging preferably between 1 and 2.0;
- from 5 to 35 parts of bis(alpha-t-butyl-peroxy-isopropyl)-benzene (II), the two substituents being in meta and/or para position with respect to the benzene ring, the meta/para ratio ranging preferably between 1.0 and 2.0;
- from 30 to 55 parts by weight of alpha(t-butyl-peroxy-isopropyl)-alpha -(t-amyl-peroxy-isopropyl)-benzene (III), the two substituents being in meta and/or para position, the meta/para ratio ranging preferably from 1 and 2.0;
provided that when components (I), (II) and (III) are contemporaneously in the para form, (I) is comprised between 30 and 55 and (II) is comprised between 5 and 15.

2. Liquid peroxidic compositions according to claim 1, wherein (I), (II) and (III) are in the form of meta/para isomeric mixtures.

3. Use of the liquid peroxidic compositions of claims 1-2 in the vulcanization of polymers.

4. Use of the liquid peroxidic compositions according to claim 3, wherein the polymer is selected among polyethylene, ethylene-propylene elastomers, eventually containing a dienic monomer.

5. Process for the preparation of the liquid peroxidic compositions according to claims 1-2 wherein the benzene is alkylated in the presence of AlCl₃ according to the Friedel-Kraft reaction, the obtained diisopropylbenzene, eventually rectified to separate meta and para isomers, is oxidized till the dihydroperoxide of diisopropylbenzene is obtained, its successive extraction and reduction in an alcaline environment to obtain the dialcohol: 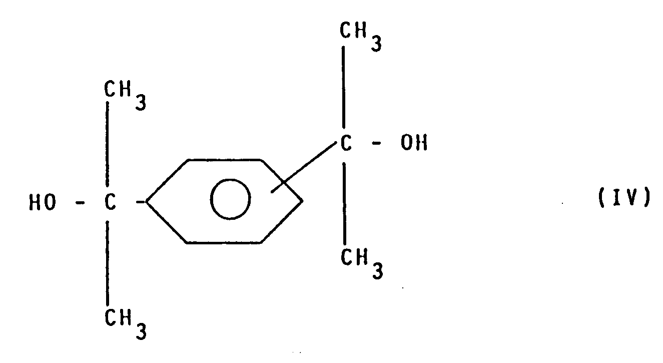 the two groups being in meta and/or para position; separation of the alcohol from the aqueous phase, reaction of the dialcohol with tert.butyl and tert.amyl hydroperoxide in the ratio from 3:2 to 1:4 in the presence of acid catalysts, so as to obtain components I, II and III; mixture washing with alkaline solution and elimination of the low-boiling products.

6. Process according to claim 5, wherein the two tert.butyl and tert.amyl hydroperoxides are simultaneously prepared by reaction of tert.butyl alcohol, tert.amyl alcohol, or isobutylene, isopentene in the presence of a hydrocarbon solvent, hydrogen peroxide, operating in acid environment and successive elimination of the aqueous phase.

7. Bisperoxide of formula: 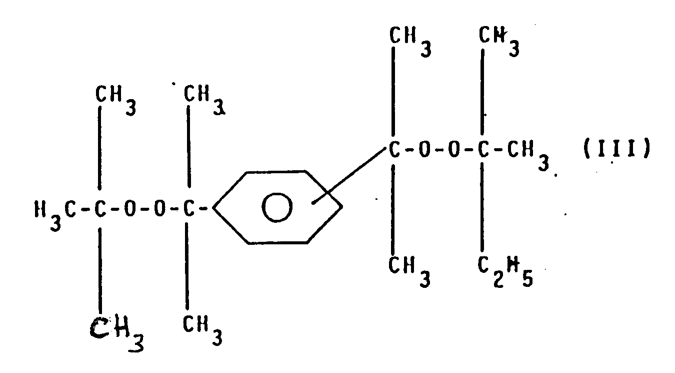 the two substituents being in meta and/or para position with respect to the benzene ring, the meta/para ratio ranging from 1 and 2.0.

## Patentansprüche

1. Flüssige peroxidische Zusammensetzungen, welche aus folgenden Bestandteilen pro 100 Gewichtsteile der Zusammensetzung bestehen:
- 10 bis 55 Gewichtsteile Bis(alpha-t-amylperoxy-isopropyl)-benzol (I), wobei die beiden Substituenten in der meta- und/oder para-Position des Benzolringes sind und das meta/para Verhältnis vorzugsweise zwischen 1 und 2,0 liegt;
- 5 bis 35 Teile Bis(alpha-t-butyl-peroxy-isopropyl)-benzol (II), wobei die beiden Substituenten in der meta- und/oder para-Position des Benzolringes sind und das meta/para-Verhältnis vorzugsweise zwischen 1,0 und 2,0 liegt;
- 30 bis 55 Gewichtsteile alpha(t-Butyl-peroxy-isopropyl)-alpha-(t-amyl-peroxy-isopropyl)-benzol (III), wobei die beiden Substituenten in der meta- und/oder para-Position sind und das meta/para-Verhältnis vorzugsweise zwischen 1 und 2,0 liegt;
vorausgesetzt, daß, wenn die Bestandteile (I), (II) und (III) gleichzeitig in der para-Form vorliegen, (I) zwischen 30 und 55 umfaßt und (II) zwischen 5 und 15.

2. Flüssige peroxidische Zusammensetzungen nach dem Anspruch 1, worin (I), (II) und (III) in Form von meta/para-Isomergemischen vorliegen.

3. Verwendung der flüssigen peroxidischen Zusammensetzungen nach den Ansprüchen 1-2 bei der Vulkanisierung von Polymeren.

4. Verwendung der flüssigen peroxidischen Zusammensetzungen nach Anspruch 3, wobei das Polymere ausgewählt wird aus Polyethylen, Ethylen-Propylen-Elastomeren, welche gegebenenfalls ein Dien-Monomeres enthalten.

5. Verfahren zur Herstellung der flüssigen peroxidischen Zusammensetzungen nach den Ansprüche 1-2, wobei das Benzol in Gegenwart von AlCl₃ nach der Friedel-Kraft-Reaktion alkyliert wird, das erhaltene Diisopropylbenzol, gegebenenfalls rektifiziert, um die meta- und para-Isomeren zu trennen, oxidiert wird, bis das Dihydroperoxid von Diisopropylbenzol erhalten wird, dessen nachfolgende Extraktion und Reduktion in einer alkalischen Umgebung, um den Dialkohol: 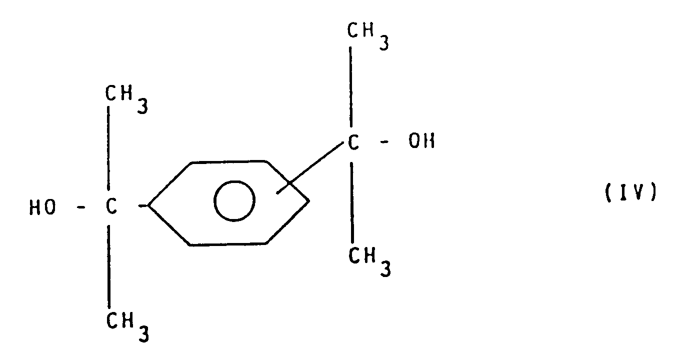 zu erhalten, wobei die beiden Gruppen in der meta- und/oder para-Position sind; Abtrennung des Alkohols von der wässrigen Phase, Reaktion des Dialkohols mit tert.-Butyl- und tert.-Amylhydroperoxid im Verhältnis von 3:2 bis 1:4 in Gegenwart von Säurekatalysatoren, um die Bestandteile I, II und III zu erhalten; Waschen des Gemisches mit einer alkalischen Lösung und Abtrennung der niedrig siedenden Produkte.

6. Verfahren nach Anspruch 5, wobei die beiden tert.-Butyl- und tert.-Amylhydroperoxide gleichzeitig durch Reaktion von tert.-Butylalkohol, tert.-Amylalkohol oder Isobutylen, Isopenten in Gegenwart eines Kohlenwasserstofflösungsmittels mit Wasserstoffperoxid erhalten werden, wobei in einer sauren Umgebung gearbeitet wird, und anschließende Entfernung der wässrigen Phase.

7. Bisperoxid der Formel: 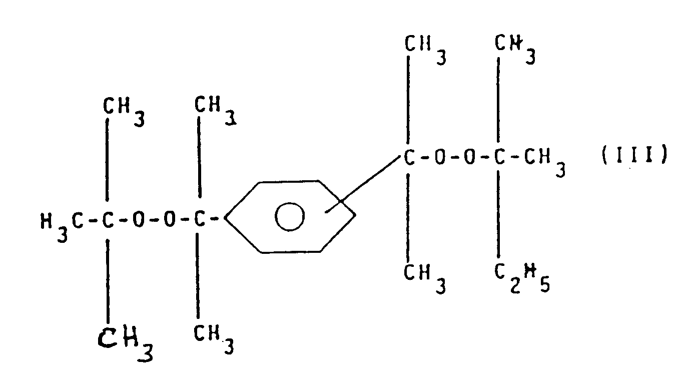 wobei die beiden Substituenten in der meta- und/oder para-Position im Benzolring vorliegen und das meta/para-Verhältnis zwischen 1 und 2,0 beträgt.

## Revendications

1. Compositions peroxydiques liquides, constituées par les composants suivants, pour 100 parties en poids de composition :
- de 10 à 55 parties en poids de bis(alpha-t-amyl-peroxy-isopropyl)-benzène (I), les deux substituants étant en position méta et/ou para par rapport au noyau benzène, le rapport méta/para se situant, de préférence, entre 1 et 2,0 ;
- de 5 à 35 parties de bis(alpha-t-butyl-peroxy-isopropyl)-benzène (II), les deux substituants étant en position méta et/ou para par rapport au noyau benzène, le rapport méta/para se situant, de préférence, entre 1,0 et 2,0 ;
- de 30 à 55 parties en poids d'alpha(t-butyl-peroxy-isopropyl)-alpha-(t-amyl-peroxy-isopropyl)-benzène (III), les deux substituants étant en position méta et/ou para, le rapport méta/para se situant, de préférence, entre 1 et 2,0 ;
à la condition que, lorsque les composants (I), (II) et (III) sont simultanément dans la forme para, (I) est compris entre 30 et 55, et (II) est compris entre 5 et 15.

2. Compositions peroxydiques liquides selon la revendication 1, dans lesquelles (I), (II) et (III) sont dans la forme de mélanges isomères méta/para.

3. Utilisation des compositions peroxydiques liquides, telles que définies à l'une des revendications 1 et 2, dans la vulcanisation de polymères.

4. Utilisation des compositions peroxydiques liquides selon la revendication 3, dans laquelle le polymère est choisi parmi le polyéthylène, les élastomères éthylène-propylène, le cas échéant contenant un monomère diénique.

5. Procédé de préparation des compositions peroxydiques liquides telles que définies à l'une des revendications 1 et 2, dans lequel le benzène est alkylé en présence d'AlCl₃ conformément à la réaction de Friedel-Kraft, le diisopropylbenzène obtenu, le cas échéant rectifié pour séparer les isomères méta et para, est oxydé jusqu'à ce que le dihydroperoxyde de diisopropylbenzène soit obtenu, son extraction et sa réduction successives dans un environnement alcalin pour obtenir le dialcool : 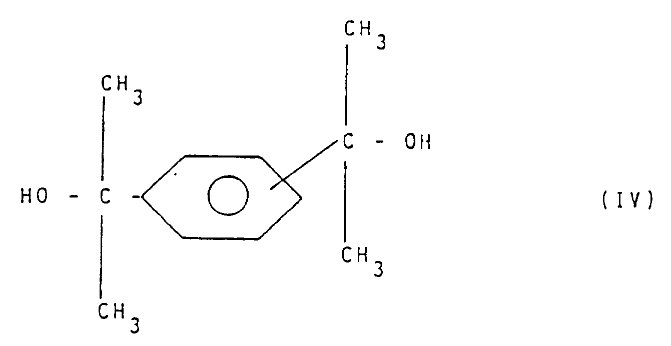 les deux groupes étant en position méta et/ou para ; séparation de l'alcool à partir de la phase aqueuse, réaction du dialcool avec les hydroperoxydes de tert.-butyle et de tert.-amyle dans le rapport de 3:2 à 1:4 en présence de catalyseurs acides, de façon à obtenir les composants (I), (II) et (III) ; lavage du mélange avec une solution alcaline et élimination des produits à faible point d'ébullition.

6. Procédé selon la revendication 5, dans lequel les deux hydroperoxydes de tert.-butyle et de tert.-amyle sont simultanément préparés par réaction d'alcool tert.-butylique, d'alcool tert.-amylique ou d'isobutylène, d'isopentène en présence d'un solvant hydrocarboné, de peroxyde d'hydrogène, en opérant dans un environnement acide, et élimination successive de la phase aqueuse.

7. Bisperoxyde de formule : 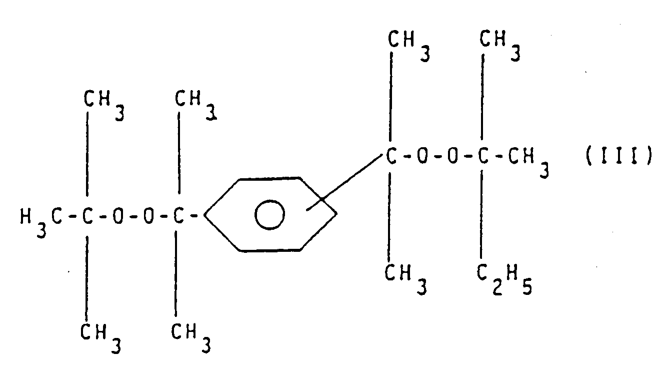 les deux substituants étant en position méta et/ou para par rapport au noyau benzène, le rapport méta/para se situant entre 1 et 2,0.
